# EUROPEAN PATENT SPECIFICATION

(11) **EP 3 720 356 B1**
(45) Date of publication and mention of the grant of the patent: **09.10.2024**
(21) Application number: 17823058.7
(22) Date of filing: 08.12.2017
(51) Int. Cl.: A61B 8/00, A61N 7/02, A61B 18/00, A61N 7/00, A61B 90/00

(54) **ULTRASOUND DEVICE**
ULTRASCHALLVORRICHTUNG
DISPOSITIF À ULTRASONS

(43) Date of publication of application: 14.10.2020
(73) Proprietor: Theraclion SA, 92240 Malakoff (FR)
(72) Inventor: GRISEY, Anthony, 78210 Saint Cyr l'Ecole (FR); YON, Sylvain, 92220 Bagneux (FR); ANQUEZ, Jérémie, 75018 Paris (FR); LEVEAU-MOLLIER, Sévérine, 91570 Bièvres (FR)
(74) Representative: Hepp Wenger Ryffel AG
(86) International application number: PCT/EP2017/082107
(87) International publication number: WO 2019/110133

(56) References cited:
- EP-A1- 2 886 160
- US-A1- 2005 065 531
- US-A1- 2007 088 346
- US-B1- 6 206 843
- US-B1- 6 306 130

## Description

The invention relates to an ultrasound device and a method for operating an ultrasound device, in particular a HIFU device, according to the independent claims.

Ultrasound devices and in particular HIFU (High Intensity Focussed Ultrasound) devices are well known in the art. An example of such a device is described in EP 2 504 063 of the applicant. Ultrasound device are commonly used for the non-invasive treatment of tissue, mainly based on two effects, thermal ablation and cavitation. EP2886160, US6306130, US2005/065531 and US2007/088346 disclose further prior art relevant for the present invention.

According to the general use of ultrasound and in particular HI-FU devices, the probe head is approximately placed over the object (i.e. a patient) to be treated. Then precise positioning of the focus with respect to the target is done under imaging guidance provided by the associated imaging device (B-mode, MRI, etc.) with mechanical motion of the probe head (e.g. if mounted on a robot) or using electronic focusing. Generally, the probe head contains a coupling liquid which ensures the acoustic coupling to the object. To contain the coupling liquid, the probe head is closed by a coupling balloon on its distal end. The coupling liquid may be circulated by a set of pumps. Since the balloon is in contact with the object, part of the fluid pressure is transmitted to the object. In this context, the balloon and the fluid pressure play an important role in the targeting: a change in the fluid pressure in the balloon will tend to offset the focus away from the target. High pressure will tend to compress a soft object, not only the superficial layers but also to certain extent the deeper structures. For example, by applying pressure to a tissue of a patient by means of the coupling liquid pressure inside the balloon and/or by pressing the probe head with the balloon against the tissue, an underlying vein may be partially or totally collapsed.

In order to maintain a high targeting accuracy, some systems control the pressure of the fluid within the balloon.

For example, a "constant pressure system" (i.e. capable of maintaining a constant pressure in the balloon) uses a pressure sensor in the probe head. A controller unit with a feedback loop which receives information from the pressure sensor acts on a fluid control system so as to ensure a constant pressure of the coupling liquid in the balloon. This enables the device to comply with the object characteristics such as shape, size, contact surface with the balloon, etc. by maintaining contact when the probe head is moved away from the object by increasing the coupling liquid volume within the balloon (inflation) and by avoiding too high mechanical stress on the object when the treatment head is moved towards the object by decreasing the fluid volume within the balloon (deflation) or by applying a constant pressure on the object (e.g. for partially or totally collapsing a vein). In other systems, the volume of the fluid is kept constant.

In the course of these adjustments by an operator, a "constant pressure system" will adjust the quantity of coupling liquid in the balloon to maintain a constant pressure of the balloon onto the object. However, a delay is introduced by the feedback mechanism. For example, the length of the tubes in which the coupling liquid flows may causes a delay in the increase of pressure in the balloon. Accordingly, it can take up to a few seconds for the balloon to reach the set pressure value.

In the context of the operation, this leads to the following unwanted sequence of events: In a first phase, the operator manually compresses and/or moves the object (i.e. a patient tissue) or the probe head to bring the target onto focus. The pressure feedback loop detects an abrupt increase in fluid pressure, and starts deflating the balloon. The beginning of the deflation is quite quick due to the high pressure value reached, which enables the operator to place the target at focus without too much effort.

However, when the operator reaches proper positioning, the pressure has not yet fully reached its set value. Therefore, in the following seconds, the balloon slowly deflates and the target slowly drifts away from the focus. The operator would generally compensate that drift manually, forcing the operator to maintain its potentially uncomfortable position for several seconds and slowing down the procedure.

Although those phases are described separately to illustrate the feeling of the operator, in reality, there is a continuous shift from the fast adjustment phase to the slow adjustment phase along with the decrease (or increase) in pressure towards its set value.

Increasing the gain parameter in the feedback loop makes the system more reactive to pressure changes but can lead to instable regimes where the balloon is continuously inflating and deflating, thus leading to an unwanted oscillation of the focus position.

According to existing techniques for the treatment of veins as disclosed in US 7,921,854 B2 or US 6,752,803 B2, a tourniquet for reducing or stopping the blood flow upstream of the target area is used. Other techniques comprise placing the patient in the Trendelenburg position in order to decrease the blood flow or tumescent anesthesia, where liquid is injected around the target area (preferably in a fascia surrounding the vein), which reduces the vein diameter. Mechanical compression with bandages is also used.

When a vein is collapsed, the problem arises that the vein is not visible anymore in commonly used imaging devices. For example, a fully collapsed vein is not visible using B-mode ultrasound imaging. Similarly, in colour Doppler imaging, stopping the blood flow will render the vein downstream (that means the vein without blood stream) invisible, even if the vein is not collapsed.

It is therefore an aim of the present invention to provide an ultrasound device for the treatment of veins, which avoids the drawbacks of the prior art and in particular allows reliable and fast variation of the compression of the object necessary to optimally perform different tasks/steps during operation.

This aim is solved by a device according to the independent claim of the present invention.

According to the present invention, an ultrasound treatment device, in particular a HIFU treatment device comprises a probe head comprising an ultrasound transducer for delivering treatment ultrasound waves focused onto a target within an object. The device includes an imaging device for imaging of the object.

The device further comprises a compression device for applying a compression force to at least some parts of the object located near the target and the probe head an in particular to apply a compression force to the target area to be treated.

As it will become more clear from the description below, the compression device may be in a preferred embodiment the balloon itself, where the pressure of the coupling liquid is used to apply a compression force to the object. Alternatively, the compression device may be a moving mechanism of the probe head, where the probe head is pressed towards the object to apply a compression force (e.g. by clamping the object between the probe head and an abutment). The compression device may also be a separated device, in particular attached to the probe head and arranged near the contact point of the balloon with the object, which can be moved and or inflated/deflated in order to apply a compression force to the object.

Further, a controller unit the probe head and the compression device is present.

The device is adapted to switch between at least two operating modes.

In a first operating mode the compression force applied to the object by the compression means is maintained at a first compression force value.

In a second operating mode the compression force applied to the object by the compression unit is maintained at a second compression force value which is higher than the first compression force value.

The compression force is in particular induced by a pressure or a volume of a coupling liquid arranged in a cavity of a coupling balloon of a probe head.

A coupling balloon may be arranged at a target side of the probe head, wherein a cavity between the probe head and the coupling balloon is filled or fillable with a coupling liquid. The balloon may be a deformable balloon.

The cavity of the balloon is preferably in fluid connection with a coupling fluid reservoir.

The device may further comprise a pressure measurement unit for determining the pressure of the coupling liquid in the cavity, and a fluid control system for controlling the pressure or the volume of the coupling liquid in the cavity.

The pressure measurement unit preferably comprises a pressure sensor. Alternatively, the pressure may be determined using other criteria such as the volume of coupling liquid in the cavity or in a part/subsection of the cavity (taking the density of the coupling liquid into consideration and using gravity).

The fluid control system may preferably comprise a pump and at least one valve or at least two pumps. Coupling liquid can therefore be circulated within the balloon and in particular between the balloon and the fluid reservoir. The coupling liquid may be additionally kept to a constant temperature, in particular cooled by means of a heat exchanger.

In the first operating mode, the controller unit controls the device in order to perform imaging of the object, while in the second operating mode, in particular when the second compression force is higher than the first compression force, the controller unit controls the device in order to emit at least one pulse of treatment ultrasound waves.

The controller unit is connected at least to the pressure measuring unit, the fluid control system, the probe head and the compression device.

In the first operating mode the pressure of the coupling liquid is maintained at a first pressure value and/or maintained at a first volume value and/or controlled according to a first control rule.

In the second operating mode the pressure of the coupling liquid is maintained at a second pressure value, preferably higher than the first pressure value or the volume of the liquid in the cavity is kept constant at a second volume value and/or the pressure is controlled according to a second control rule different from the first control rule. As an example, in the second operating mode, if a fluid control system comprising two pumps is used, the speed of the pumps may be controlled by the controller unit, in particular to keep the volume constant to the second volume value. It must be noted that in this case, if the speed of the pumps is equal, a slight drift in the volume value may be exist and may be compensated by adjusting the speed of one of the pumps from time to time.

A control rule is in particular a defined control loop mechanism. The first control rule may e.g. differ from the second control rule in that the pressure is controlled in a more delayed way as according to the second rule.

The first operating mode is a imaging mode and preferably a positioning mode. In particular, the first pressure value or the first volume value is suitable for the tasks of positioning the probe head onto the object and/or imaging the object and in particular the target area of the object and/or roughly focusing the probe head on the target.

The second operating mode is a treatment mode. In particular, the second pressure value or the second volume value is suitable for the tasks of displacing the object, in particular the soft tissue of a patient, in order to bring and keep focus into the target area and/or treating the target by means of emitting at least one pulse of treatment ultrasound waves.

In this regard, the second operating mode is adapted for partial or total collapsing of a vein and/or stop blood flow for the treatment of varicose veins. Partial collapsing according to the present invention is defined as the lumen of the vein still visible by means of the imaging device, wherein the lumen section area is smaller than a reference (i.e. when a patient is standing) wherein totally collapsing of the vein is defined by the vein being totally compressed and therefore the lumen of the vein being not more visible by means of the imaging device.

For the treatment of veins, both thermal and cavitation effects can be used, alone or in combination. In particular, two main principles are used, namely stopping the blood flow and/or collapsing (partially or totally) the vein.

Stopping blood flow mainly aims at avoiding its cooling effect on the vein wall. Collapsing the vein can bring more of the vein wall within the focus, thus considerably decreasing treatment time and/or increasing treatment efficacy.

Stopping blood flow can be done by compressing the vein distally or proximally to the targeted zone. A distal compression may however not be efficient since, if a tributary branch to the vein is present between the compression point and the targeted area, it will bring some flow to the vein. Conversely, proximal compression is more efficient in stopping the flow but blood will tend to accumulate within the vein, thus inflating it. Mechanical compression just above the targeted area is difficult to perform with existing devices.

Alternatively, ultrasound emission may be performed with some blood still present in the vein (partial collapsing of the vein) in order to treat the vein wall by cavitation.

It must be noted at this point that according to this preferred embodiment, if in the first operating mode the pressure is maintained to the first pressure value, in the second operating mode the controlled parameter must not necessarily be the pressure, but may also be the volume (second volume value) and vice-versa.

The controller unit is preferably adapted to monitor the pressure of the coupling liquid determined by the pressure measurement unit and to detect a change and in particular an increase of the pressure of the coupling liquid in the cavity when the device is in the first operating mode and the coupling balloon is arranged on the object.

The controller unit is further preferably adapted to switch from the first operating mode to the second operating mode upon detection of a switch signal.

In particular, if a compression force is applied to the object by the compression device or by an operator, in the second mode, the balloon is not, as known from the prior art, deflated in order to keep the pressure constant, but is only deflated to a second pressure value higher than the first pressure value or the volume is set and then kept constant to a second volume value in order to avoid the slow shifting of the target position during the slow adjustment phase as described in the introduction.

When the device is in the second operating mode, the device triggers the emission of at least one pulse of treatment ultrasound waves and in particular of a treatment sequence comprising a plurality of treatment ultrasound wave pulses.

Preferably, the balloon may be shaped such that deformation of the balloon mainly occurs in a direction toward the focus. This may be achieved by providing a balloon with lateral stiff walls and a flexible membrane on the part facing the ultrasound transducer. Alternatively, cover members may be applied to conventional balloons in order to constrain lateral deformation. Therefore, the pressure of the coupling liquid may be better controlled when applying pressure to the object.

The device is further adapted to switch back from the second operating mode to the first operating mode after the emission of at least one pulse of treatment ultrasound waves.

The device may preferably comprise a manual switch for switching between the first operating mode and the second operating mode. In this case, the switching signal is generated by actuating the manual switch.

Preferably, a delay is introduced between activation of the manual switch and generation of the switching signal in order to allow an operator to perform other tasks, e.g. compress the object manually or by means of the compression device.

In particular, in the cases where switching from the first operating mode to the second operating mode is automatic, as compression of the tissue is complex and requires several trials to find the right position of the hands or of the compression device, the operator has the possibility to switch to a "training mode" where he can freely test the compression without generating the switching signal, while checking the effect of his compression by means of the imaging device.

Optionally, when the operator is satisfied by the position of his hands, the operator can switch to a "learning mode", where the operator successively performs his compression manoeuvre one to several times (preferably, the number of compression manoeuvres is pre-defined). During this phase, the device analyses the pressure curve in order to determine the appropriate first pressure threshold value. For example, if the operator performs the compression one time, the first threshold value can be set to e.g. 80% of the maximum pressure reached.

A manual switch according to the present invention must not necessarily be activated by the hand of an operator, but can also be voice activated or a foot or a leg switch.

Alternatively, the controller unit is further adapted to switch from the first operating mode to the second operating mode when the detected pressure increase is above a threshold value. In this case, the switching signal is generated automatically by the controller unit when the detected pressure increase is above the threshold value. This is in particular advantageous when the object compression is performed with the compression device or manually by an operator.

Preferably, the threshold value is greater (e.g. tenfold) than the standard variation of the pressure value measured during the first operating mode to avoid untimely switching. Other control terms such as the rate of change of the pressure value (D-term) may be used for generating the switching signal and therefore switch the operating mode from the first to the second operating mode.

Preferably, if the controller unit is further adapted to switch from the first operating mode to the second operating mode when the detected pressure increase is above a threshold value, the switching signal is generated with a delay.

In particular, after the pressure value has reached a value above the first threshold value, the controller unit first controls the pressure in the balloon in order to lower it to the second pressure value. When the pressure in the balloon has reached a second pressure threshold value and drops below this second pressure threshold value, the switching signal is generated.

Preferably the controller unit is further adapted to switch from the second operating mode to the first operating mode when the detected pressure decrease is below a pressure threshold value. In this case, the switching signal is generated automatically by the controller unit when the detected pressure decrease is below the pressure threshold value. This is in particular advantageous when the object compression is performed with the compression device or manually by an operator and the compression is lowered after the emission of the ultrasound treatment waves.

The device preferably further comprises at least one force sensor, preferably a finger force sensor for a device operator, connected to the controller unit and preferably arranged or arrangeable on the object, and the controller unit is further adapted to compare a force determined by the force sensor with a force threshold value and to switch the device to the second operating mode when the force determined by the force sensor is above the force threshold value and is preferably kept constant for a pre-determined amount of time.

The force sensor may be arranged on a glove worn by an operator. Alternatively, the force sensor may be adapted to determine a compression force applied by the compression device to the object.

The force sensor is preferably used for detecting the force applied by an operator or by the compression device onto the object, which object may be soft and deformable. Therewith it may be possible to determine if the operator or the compression device is trying to keep the target in the focus zone of the probe head. Switching to the second mode is preferably performed when the force measured remains stable at a high value for a short duration compared to a characteristic time of the slow adjustment phase (typically, from 0.1 to 1 seconds).

The controller unit is further preferably adapted to switch from the second operating mode to the first operating mode after the emission of at least one pulse of treatment ultrasound waves when the force determined by the force sensor is below a second threshold value and preferably remains constant for a pre-determined amount of time.

In particular, the force measurement may be used to abort the emission of ultrasounds by just decreasing the force applied to the object (e.g. by removing the hands from the object or reducing the compression force applied to the object).

The controller unit is further preferably adapted to automatically trigger the emission of and emit at least one pulse of treatment ultrasound waves after activation of the second operating mode.

In this preferred embodiment, the device is able to determine that the target has been correctly located within a focus of the probe head and therefore an emission of ultrasounds can be automatically performed.

It must be noted at this point that throughout the present description, the terms "triggering the emission of at least one pulse of treatment ultrasound waves" and "emitting at least one pulse of treatment ultrasound waves" art used. Triggering the emission of at least one pulse of treatment ultrasound waves means that the device is in a state where he is actually ready to emit the treatment ultrasound waves, while the emission of the waves may be delayed. In particular, the emission of the treatment ultrasound waves is performed only if supplementary criteria are met.

Alternatively, the device may further comprise a manual switch for triggering the emission of at least one pulse of treatment ultrasound waves by an operator, wherein switching from the first operating mode to the second operating mode is performed automatically after triggering the emission of at least one pulse of treatment ultrasound waves, in particular if compression of the object is to be performed by means of the balloon.

According to this preferred embodiment, the operator can agree with the device emitting the ultrasounds before the target has been properly focused. The device can then automatically switch from the first operating mode to the second operating mode as described in the present application. Interruption of the procedure may be possible at any time (e.g. by removing the hands from the object).

In order to correctly place the focus with respect the target, the device preferably further comprises time measuring means for delaying the emission of at least one pulse of treatment ultrasound waves by a pre-determined amount of time after the second operating mode has been activated.

According to this preferred embodiment, the emission of ultrasounds is delayed in order to allow an operator to focus the target. The pre-determined amount of time may be chosen by the operator manually or may be determined automatically based on operator's related data such as the time normally needed for focusing the target according to an activity register etc.

The time measuring means may be preferably further adapted to automatically switch from the second operating mode to the first operating mode with a delay of a pre-determined amount of time after emission of the treatment ultrasound waves.

The controller unit is preferably further adapted to determine the position of the target based on the images captured by the imaging device and to switch to the second operating mode automatically when the position of the target is stable over a pre-determined amount of time.

As cited above, imaging of the target is usually performed in order to properly focus the target. Known image processing algorithms may be used to determine if the target position is kept within an allowable target zone for a pre-determined amount of time.

The controller unit may be further adapted to switch to at least a third operating mode where the compression force applied to the object by the compression unit is maintained at a third compression force value, preferably higher than the second compression force value, and in particular the pressure of the coupling liquid is maintained at a third pressure value, preferably higher than the second pressure value, or the volume of the coupling liquid in the cavity is maintained at a third volume value.

Switching to the third mode is preferably performed just before and during ultrasound emission, to apply a higher pressure to the vein to further reduce the vein section or totally collapse the vein.

Also in this case it must be noted that according to this preferred embodiment, if in the first or second operating mode the pressure is maintained to the first or second pressure value, in the third operating mode the controlled parameter must not necessarily be the pressure, but may also be the volume (third volume value) and vice-versa.

Preferably, the controller unit is adapted to switch from the third operating mode to the second operating mode during treatment ultrasound emission, such that at least one pulse of treatment ultrasound waves is emitted with a vein totally collapsed and at least one pulse of treatment ultrasound waves is emitted with the vein in a partially collapsed.

After the emission of the treatment ultrasound waves, the controlling unit is preferably adapted to reduce the pressure of the coupling liquid in the cavity so that vein may be visible again by means of the imaging device.

Therefore, the device may be switched back to the first or to the second operating mode.

The controller unit may then be adapted to move the focus to the next location to be treated. Alternatively, the operator may move the focus to the next location.

Alternatively, the focus may be moved to the next location and the device may be then switched back to the first or second operating mode.

Compression of the vein may be also maintained during a sequence of ultrasound pulses before compression is released: for example, if several pulses have to be delivered in the same transverse plane and the displacements are small (e.g. < 5 mm). Preferably, in the first operating mode particularly adapted for probe head positioning and imaging, a slight pressure is applied to the object (e.g. 0.5-15 mbar).

In the second operating mode, which is particularly adapted for targeting (and potentially for cavitation pulses) a moderate pressure, causing partial collapse of the vein, is applied (e.g. 5-40 mbar).

In the third operating mode, which is particularly adapted for pulses relying on thermal effects, a higher pressure causing complete collapse of the vein is applied (e.g. 30-200 mbar).

It must be noted that in the different operating modes, other parameters may be changed by the controller unit. In particular, in the case of a proportional feedback loop, increasing the pressure value to be maintained may require to decrease the gain of the feedback loop to avoid oscillations.

Preferably, the control unit further comprises an input device for manually inputting parameters related to the different operating modes. In particular, the pressure values to be maintained during the respective operating modes may be adjusted by an operator. In addition, the rate of pressure change when switching from one operating mode to the other operating mode may be variated by an operator. In particular, when switching from the first operating mode to the second operating mode and from the second operating mode to the third operating mode, the rate of pressure change is chosen such as to allow an operator and/or an automatic segmentation algorithm implemented in the controller unit to follow a change in the object, e.g. to determine if a vein is partially or totally collapsed.

Compression of the object, especially if it is performed with the balloon, induces some displacement of the object and of the target. Therefore, if the focus was placed on the target before compression, it is likely to be at a wrong position after compression.

In the case of compression with the balloon, it may be possible to anticipate how the object will deform, in particular how a vein will collapse, due to compression. Therefore, the focus may be placed slightly away from the target with reference to the probe head such that after compression, the deformed object is at focus.

As an example, positioning the focus 5 mm under the target with a pressure of 5 mbar in the balloon usually results in the target being displaced approximately to the focus when a pressure of 50 mbar is applied.

In particular, the controller unit may be adapted to provide a marker on an image provided by the imaging device indicating the position where the target is presumed to be located after a compression is applied to the object. This is in particular done by the controller unit being connected or comprising a computing unit with operational instructions stored therein for providing the marker. Preferably, the marker is only displayed during the first operating mode.

The focus positioning may be also performed by means of a trial and error procedure. The device is adapted to increase the pressure of the balloon to a pressure of the third operating mode. Imaging of the object is performed until the target disappears from the image or reaches a pre-determined level of collapsing (e.g. at 60 mbar), e.g. blood flow has been stopped as assessed by colour Doppler or a vein lumen section area assessed by B-mode imaging has reached a desired value.

At the end of this phase, the target may or may not be visible by means of the imaging device. Therefore, especially if it is intended to become invisible at the end of the phase, the position of the targeted object is followed along compression. Preferably, a tracking algorithm is used to follow its position as it collapses. Most preferably, its position is still automatically followed after it has become invisible (e.g. based on its last visible position and a speckle tracking algorithm). Alternatively, the operator visually follows its position as it collapses.

Then, if the target in the (partial or total) collapsed state is not at the desired position (e.g. with the focus within the vein lumen), the device is moved by the offset distance to bring the focus on target.

Optionally, the device is further switched to the second operating mode where the target is visible by means of the imaging device. The position of the focus with respect to the target is then determined and, if the focus is offset, the probe head is moved along the imaging plane. The pressure may be decreased when moving the probe head (the device is switched to the first operating mode).The procedure is repeated until the focus at the desired location with respect to the target.

As cited above, when the object is compressed, it may be impossible to see the target by means of the imaging device, e.g. when no blood is flowing through the vein.

By moving the probe head away from the target for a short period of time, this problem can be solved.

The device therefore comprises means for moving the probe head away from the object. The movement shall be of a short duration of time, such that the balloon is not inflated due to the detected fast pressure decrease, and may be in the range of some millimetres.

Alternatively, the probe head may be displaced manually by an operator to obtain the same results.

Imaging is still performed when moving the probe head away from the target such that the position of the target can be identified clearly without increasing too much the blood flow in the vein.

The device is then adapted to keep the probe head at the new position (in this case the pressure would be adjusted back to the pre-determined value where the target is not visible).

Alternatively, the device may be returned to the original position. Moving the probe head away from the target and then back to the original position is in particular done in a short period of time in order not to activate pressure compensation in the balloon. In particular, moving of the probe head may be performed in a mode where the volume of the coupling liquid is kept constant by the controller unit (second operating mode).

Preferably, the controller unit is adapted to switch between a "fast-reaction mode" and a "slow-reaction mode", in particular if the probe head is manually displaced by an operator, wherein in the fast-reaction mode the pressure is adapted quickly enough when a slow movement of the probe head is done, e.g. during probe head positioning, while in the slow reaction mode, pressure is not adapted quickly when a slow movement of the probe head is done. In particular, during the "slow reaction mode" the pressure is slowly adapted such that a movement of the treatment head towards or away from the target, at a speed which enables imaging of the target, e.g. to control the opening of the vein, does not lead to significant balloon inflation or deflation. For example, the slow reaction mode can be obtained by decreasing the gain of a proportional feedback loop or by switching to a constant volume mode (which corresponds to no reaction).

In particular, the device comprises a switch for switching between the fast reaction mode and the slow reaction mode.

As an example, the controller unit may be switched to a slow reaction mode when in the third operating mode. Then, the probe head is moved away from the object (e.g. by one to a few millimetres) so the vein becomes visible. Preferably, the displacement is chosen such that a vein is only slightly opened so the operator and/or the automatic segmentation algorithm can detect it, but only limited blood flow is possible. Then, the probe head is moved back to its initial position. Finally, the controller unit is switched back to the fast reaction mode. This sequence enables to check the position of the targeted structure.

In a preferred embodiment, the ultrasound transducer may be movably arranged with respect to the balloon, wherein after determining the correct pressure for the desired treatment, e.g. the vein is collapsed for thermal ablation, the ultrasound transducer is moved with respect to the probe head and the balloon in order to shift the focus within the target without displacing the target by increasing the pressure of the liquid in the balloon because of the displacement of the probe head.

The disclosure further relates to a non-claimed method for treating a target within an object (tissue of a patient) with an ultrasound device, in particular a HIFU device. The device is preferably a device as disclosed above.

The above cited advantages and preferred embodiments of the device apply accordingly to the method according to the present invention and vice-versa.

The method comprises the following steps:
Placing a probe head comprising an ultrasound transducer for delivering treatment ultrasound waves focused onto a target within an object and applying a first compression force to the object.

In a second step, the compression force applied to the object is increased to a second compression force higher than the first compression force either manually by an operator or by means of a compression unit of the device, and at least one pulse of treatment ultrasound waves is emitted.

Preferably, imaging of the object by means of the imaging device is performed while applying the first compression force to the object.

The method preferably comprises the following steps:
Placing a deformable coupling balloon arranged at a target side of the probe head, wherein a cavity between the probe head and the coupling balloon is filled with a coupling liquid onto the object and activating a first operating mode where the pressure of the coupling liquid is maintained at a first pressure value or the volume of the liquid in the cavity is maintained at a first volume value.

Switching from the first operating mode to a second operating mode, wherein in the second operating mode the pressure of the coupling liquid is maintained to a second pressure value which is preferably higher than the first pressure value or the volume of the cavity is maintained at a second volume value.

Therefore, a compression force may be applied to the object by the coupling liquid pressure only or in addition manually by an operator and/or by means of the compression unit.

Also in this case it must be noted that according to this preferred embodiment, if in the first operating mode the pressure is maintained to the first pressure value, in the second operating mode the controlled parameter must not necessarily be the pressure, but may also be the volume (second volume value) and vice-versa.

Preferably, switching from the fist operating mode to the second operating mode is performed upon detection of a switching signal.

Preferably, the pressure of the coupling liquid is monitored by a controller unit connected to a pressure measurement unit and an increase of the pressure of the coupling liquid in the cavity is detected, wherein upon detection of the pressure increase, a switching signal is generated.

Preferably, the pressure of the coupling liquid is monitored by a controller unit connected to a pressure measurement unit an increase of the pressure of the coupling liquid in the cavity is detected, wherein the switching signal is generated if the detected pressure increase is above a pre-determined first pressure threshold value and then drops below a second threshold value, preferably within a pre-determined amount of time.

Preferably, the pressure of the coupling liquid is monitored by a controller unit connected to a pressure measurement unit and a decrease of the pressure of the coupling liquid in the cavity below a pressure threshold value is detected, wherein upon detection of the pressure decrease, a switching signal for switching the device from the second operating mode to the first operating mode is generated.

Preferably, the method comprises the step of switching to a third operating mode from the first or second operating mode, wherein in the third operating mode the pressure of the coupling liquid is maintained at a third pressure value, preferably higher than the second pressure value or the volume of the coupling liquid in the cavity is maintained at a third volume value,. Also in this case it must be noted that according to this preferred embodiment, if in the first or second operating mode the pressure is maintained to the first or second pressure value, in the third operating mode the controlled parameter must not necessarily be the pressure, but may also be the volume (third volume value) and vice-versa.

In a preferred embodiment, a third step, where at least one pulse of treatment ultrasound waves is emitted, is performed.

In a preferred embodiment, a fourth step, where the operating mode is switched from the second operating mode back to the first operating mode, is performed. This fourth step can for example be triggered by a decrease of the measured pressure below another pressure threshold value, which corresponds to the operator releasing its compression on the tissue.

In a preferred embodiment, pressure thresholds values used for triggering a switching signal would take into consideration an hysteresis of the pressure curve.

In addition, the pressure threshold values are preferably used to abort an ultrasound emission step, e.g. if the pressure of the coupling liquid raises above a pre-determined pressure value, which may be the first pressure threshold value, or sinks below another pressure value.

Preferably, imaging of the object is performed by means of the imaging device while performing the method.

Therefore, the position of the target can be monitored and the probe head may be focused with more accuracy while the method is performed.

A flow of coupling liquid between a reservoir and the cavity is preferably maintained by the pump if the device is equipped with a pump and a coupling liquid reservoir.

Preferably, the method further comprises the step of switching to a third operating mode from the first or second operating mode, wherein in the third operating mode the pressure of the coupling liquid is maintained to a third pressure value higher than the second pressure value.

In particular, the first and the second pressure values are set according to the following method:
The first pressure value is defined so as the vein being visible by means of an imaging device and the second pressure value so as the vein being in the right configuration for treatment (e.g. total collapse for thermal pulses, partial collapse for cavitation).

After arranging the probe head on the object, the pressure of the coupling liquid is gradually increased while imaging of the object is performed. After reaching the highest pressure where the vein is visible, the balloon is then deflated and the pressure of the balloon is defined as the first pressure value. Then, to define the second pressure value, the pressure is increased gradually up to the level where the desired vein shape has been reached. This pressure is then defined as the second pressure value. The pressure values are then preferably stored in a storage unit connected to the control unit and may then be selected automatically or by means of a manual switch.

The invention will now be described in more detail by way of non-limiting, exemplary embodiments in connection with the drawings which show:
- Fig. 1: schematically a device according to the present invention;
- Fig. 2A: an exemplary graph showing the coupling liquid pressure development;
- Fig. 2B: an exemplary graph showing the coupling liquid pressure development according to an alternative embodiment.
- Fig. 3A: a flow diagram of a non-claimed method; and
- Fig 3B: a flow diagram of another non-claimed method.

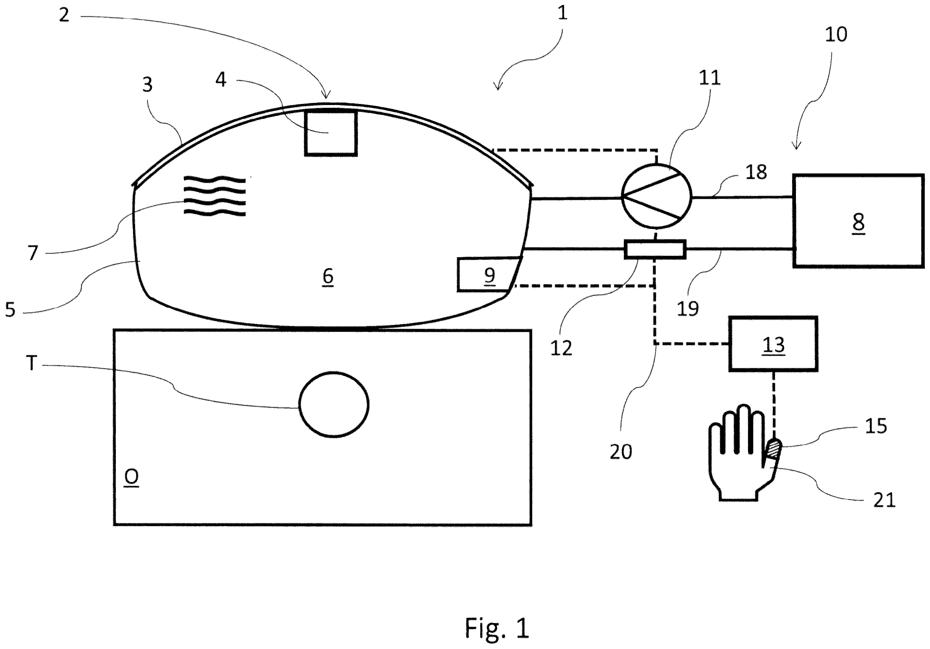

In Figure 1 there is shown an embodiment of the device 1 according to the present invention. The device 1 comprises a probe head 2 with a concave HIFU transducer 3 and a B-mode imaging device 4. The HIFU waves emitted by the HIFU transducer 3 are focused, wherein the focus lies within the imaging plane of the imaging device 4.

The device 1 further comprises a flexible balloon 5 arranged at the distal side of the probe head 2 and defining a cavity 6, which is filled with a coupling liquid 7 . The coupling liquid 7 is drawn from a reservoir 8, which is in fluid connections with the cavity 6 over lines 18 and 19. Coupling liquid is pumped into the cavity 6 by a pump 11 over the line 18 and exits the cavity via the line 19. A valve 12 for regulating the pressure inside the cavity 6 is arranged on the line 19. The pump 11 and the valve 12 therefore build a fluid control system 10 for controlling the pressure and/or the volume of the coupling liquid 7 in the cavity 6.

Inside the cavity, there is arranged a pressure sensor 9 for determining the coupling liquid pressure in the cavity 6. The HIFU transducer 2, the pressure sensor 9, the pump 11 and the valve 12 are connected to a controller unit 13 by a connection 20, which may be a wired connection such as a BUS or a wireless connection.

The controller unit 13 is adapted to control the operation of the device and is further connected to a force sensor 15 arranged on an operator's finger 21.

When the device 1 is operated, an object O is placed in contact with the balloon 5 and imaging of the object O is performed by means of the imaging device 4. A target T to be treated is located within the object O. An operator moves the probe head 2 and/or the object O such that the focus of the HIFU transducer 3 is located within the target T.

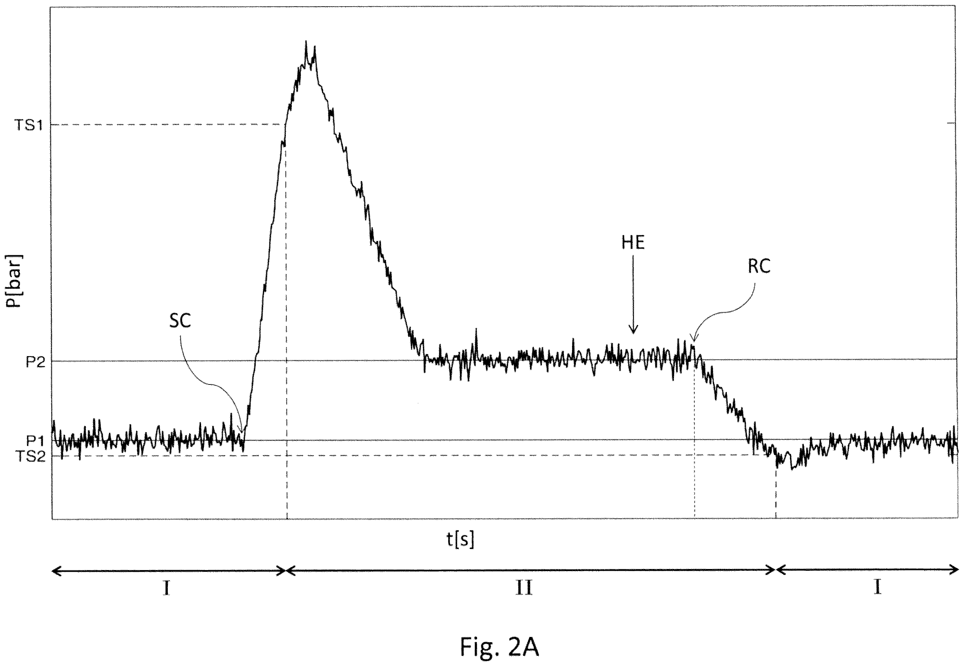

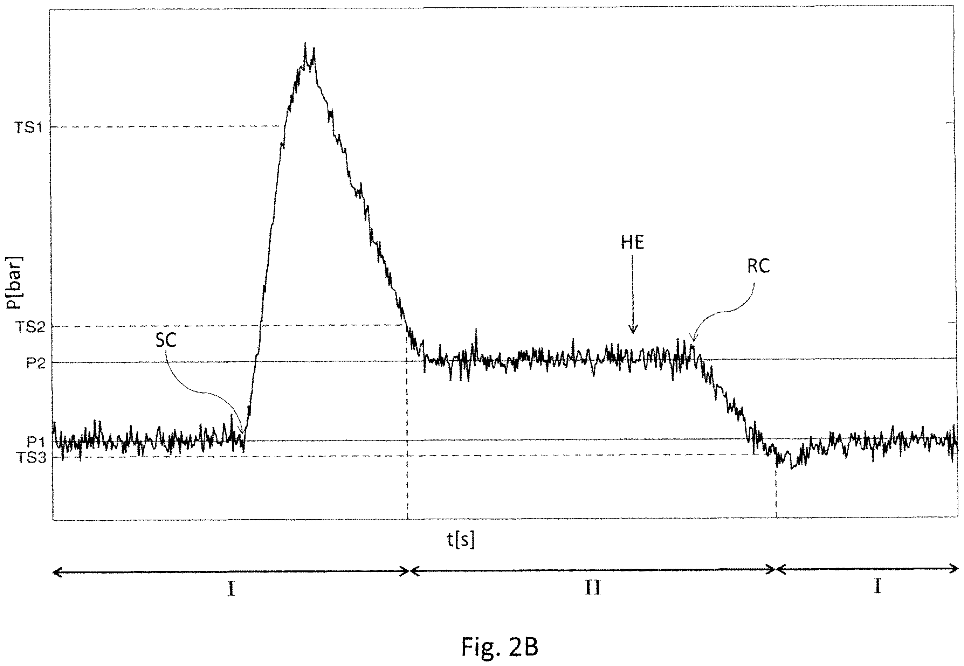

A possible curve of the pressure of the coupling liquid during operation is shown in figure 2A. The controller unit 13 keeps the pressure of the coupling liquid 7 in the cavity to a first pressure value P1. During this phase, which is also referred to as the first operating mode I (also shown schematically under the abscissa of the graph of figure 2), the probe head 2 is normally oriented such that the target T lies within the imaging plane of the imaging device 4.

In order to bring the target T in focus of the HIFU transducer 3, an operator starts compressing the object O, which is a soft tissue. Starting compression (schematically referred to in figure 2 as SC), however, leads to an undesired increase of the pressure of the coupling liquid 7.

If the pressure increase leads to a pressure value above a threshold value TS1, the controller unit 13 switches the operating mode from the first operating mode I to a second operating mode II. After switching to the second operating mode II, the controller unit 13 controls the valve 12 and the pump 11 in order to decrease the pressure to a second pressure value P2, which is higher than the first pressure value P1 kept constant during the first operating mode I.

The second pressure value P2 is then kept constant. Since the second pressure value P2 is higher than the first pressure value P1, the operator does not see the target slowly drift away from the focus as in devices known from the prior art operated with a constant pressure mode.

The emission of treatment HIFU waves is then triggered by the operator or automatically. After the emission of the HIFU waves, the operator stops compressing the object O (schematically referred to in figure 2 as RC).

The pressure drops below a threshold pressure value TS3, wherein as a response to the pressure drop under the threshold pressure value TS3, the controller unit 13 switches back to the first operating mode I, where pressure is adjusted to the first pressure value P1 and is then kept constant for the preparation of the object for the subsequent emission of HIFU waves.

An alternative of the method according to the present invention is shown in figure 2B, which is similar to the method shown in figure 2A with the difference that, when the pressure increase raises above the first threshold value TS1, the controller unit 13 does not switch directly in the second operating mode II. The pressure is controlled by the controller unit 13 and decreased. When the pressure drops below a second threshold value TS2, the controller unit 13 switches to the second operating mode II, and maintains the pressure constant to the second pressure value P2. By switching to the second operating mode II just before the pressure has reached the second pressure value P2, the operator does not see the target slowly drift away from the focus as in devices known from the prior art operated with a constant pressure mode, because the slow adjustment phase is reduced or preferably avoided.

Data from the force sensor 15 may be used additionally or alternatively in order to increase the accuracy of the detection of the pressure changes. As an example, detection of strong compression by the force sensor 15 may be used to activate the fluid control system 10 for a fast reaction of the pressure adjustment.

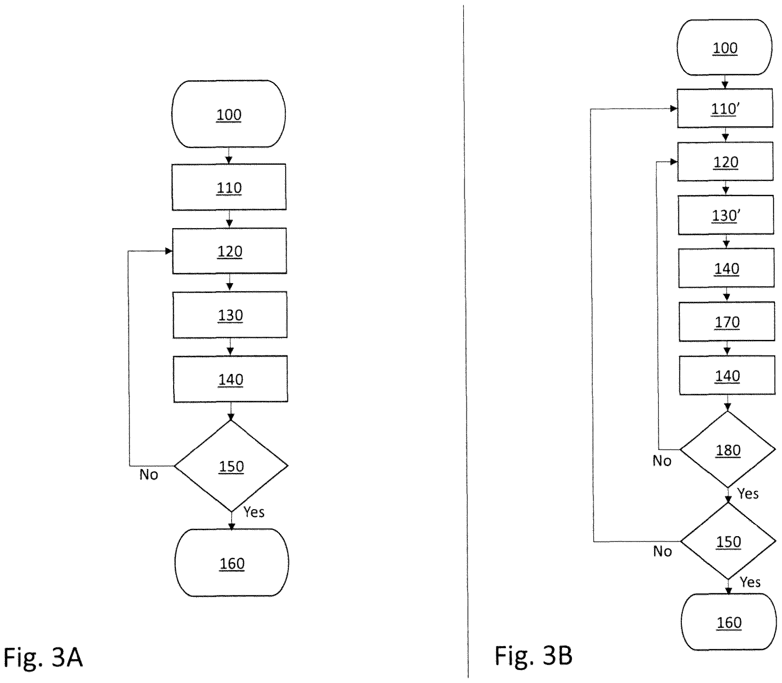

An exemplary non-claimed method is described hereinbelow with reference to the figure 3A:
The method is performed starting with a patient lying horizontally on a bed in a first step 100.

The device 1 is activated in the first operating mode I and positioned on the patient in a step 110, wherein imaging of the target T is performed by means of the imaging device 4.

The probe head 2 is then moved in a step 120 in order to roughly put the focus on the target T.

The device 1 is then switched to the second operating mode II, where the pressure P2 in the balloon is set to a value which induces a moderate compression on the tissue, which results in a partial collapse of the vein.

The probe head 2 is positioned at the centre of the vein segment to be treated in a step 130. Then, the device 1 is switched in the third operating mode III, causing the total collapse of the vein. The vein therefore becomes invisible.

The position of the focus in step 130 is adjusted based on the trial and error process described in the present invention, which implies switching between the second operating mode II and the operating mode III until the focus is at the desired position.

Then with the device in the third operating mode III, a pulse of treatment ultrasound waves is emitted in a step 140.

After the treatment ultrasound wave emission of step 140 an operator decides if the treatment is terminated or should be continued on the same target T or another target T in a step 150.

In the case the treatment is finished (YES) the method is terminated in a step 160. Otherwise (NO) the second operating mode II is activated and the pressure is decreased to the second pressure P2. The probe head 2 is then moved to the next target T according to the step 120. The method is then repeated until the desired tissue volume has been treated.

Another non-claimed method is described herein below with reference to figure 3B:
The method is performed starting with a patient lying horizontally on a bed in a first step 100.

Then the patient it is placed in the Trendelenburg position to reduce the amount of blood within the vein in a step 110' and the device 1 is activated in the first operating mode I and manually positioned on the patient, wherein imaging of the target T is performed by means of the imaging device 4. The first pressure P1 in the balloon in step 110' is set to a low value which induces minimal (if any) compression on the tissue and enables easy manual handling of the head.

The probe head 2 is then moved robotically in a step 120 in order to roughly put the focus on the target T. The device 1 head is positioned at the centre of the vein segment to be treated. The device 1 is then switched to the second operating mode II, where the pressure P2 in the balloon is set to a value which induces a moderate compression on the tissue, which results in a partial collapse of the vein.

The probe head 2 is positioned at the centre of the vein segment to be treated in a step 130'. Then, the device 1 is switched in the third operating mode III. In this case the third pressure P3 does not cause the total collapse of the vein.

The vein is totally collapsed by manual pressure application by the operator in step 130'.

The position of the focus is adjusted based on the trial and error process described in the present invention in step 130', which implies switching between the second operating mode II and the operating mode III until the focus is at the desired position.

Then, with the device in the third operating mode III and the operator manually compressing the tissue, a pulse of treatment ultrasound waves is emitted in a step 140.

After the treatment ultrasound wave emission, the second operating mode II is activated and the pressure is decreased to the second pressure P2 in a step 170.

A pulse of treatment ultrasound waves is emitted again in a step 140' with the vein partially collapsed, therefore inducing damages to the tissue by cavitation effects.

After the treatment ultrasound wave emission of step 140' an operator decides if the treatment of the target is terminated or should be continued on the same target T in a step 180.

In the case the treatment of the target T is not finished (NO), the method is performed again starting from step 120 on the same target, otherwise (YES) the operator has to decide in a step 150 if the entire treatment is terminated or not.

If the method has to be continued (NO in step 150) the method is performed again starting from step 110' by targeting a new target T. The method is then repeated until the desired tissue volume has been treated.

Otherwise, the method is terminated in a step 160.

## Claims

1. An ultrasound treatment device (1), for the treatment of veins, in particular a HIFU
treatment device, the device (1) comprising:
- a probe head (2) comprising an ultrasound transducer (3) for delivering treatment ultrasound waves focused onto a target (T) within an object (O) and an imaging device (4) for imaging of the object (O),
- a compression device for applying a compression force to the object (O), wherein the compression device is formed by one of a balloon and a moving mechanism of the probe head , and
- a controller unit (13) connected at least to the probe head (2) and the compression unit, wherein
the device (1) is adapted to switch between at least two operating modes (I, II), wherein
- in a first operating mode (I) the compression force applied to the object (O) by the compression unit is maintained at a first compression force value (CF1), and
- in a second operating mode (II) the compression force applied to the object (O) by the compression unit is maintained at a second compression force value (CF2), higher than the first compression force value (CF1), **characterized in that** in the first operating mode (I) the controller unit (13) controls the imaging device (4) to perform imaging of the object (O), and in the second operating mode (II) the controller unit (13) controls the ultrasound transducer (3) to emit at least one pulse of treatment ultrasound waves
wherein the second operating mode is adapted for partial, preferably total, collapsing of a vein and/or to stop blood flow for the treatment of the vein.

2. An ultrasound treatment device (1), according to claim 1, the device (1) comprising:
- a deformable coupling balloon (5) arranged at a target side of the probe head (2), wherein a cavity (6) between the probe head (2) and the coupling balloon (5) is filled or fillable with a coupling liquid (7), and is in particular in fluid connection with a coupling fluid reservoir (8)
- a pressure measuring unit (9) for determining the pressure of the coupling liquid (7) in the cavity (6),
- a fluid control system (10) for controlling the pressure or the volume of the coupling liquid (7) in the cavity (6), in particular comprising a pump (11) and at least one valve (12), wherein
- the controller unit (13) is connected at least to the pressure measuring unit (9), the fluid control system (10), the probe head (2) and the compression unit, and wherein
- in the first operating mode (I) the pressure of the coupling liquid (7) is maintained at a first pressure value (P1) or at a first volume value (V1), and
- in the second operating mode (II) the pressure of the coupling liquid (7) is maintained at a second pressure value (P2) higher than the first pressure value (P1) or the volume of the liquid (7) in the cavity (6) is maintained constant at a second volume value (V2).

3. Device (1) according to claim 2, wherein the controller unit (13) is adapted to:
- monitor the pressure of the coupling liquid (7) determined by the at least one pressure measurement unit (9) and to detect an increase of the pressure of the coupling liquid (7) in the cavity (6) when the device (1) is in the first operating mode (I) and the coupling balloon (5) is arranged on the object (O),
- switch from the first operating mode (I) to the second operating mode (II) upon detection of a switching signal,
- trigger the emission of at least one pulse of treatment ultrasound waves after switching from the first operating mode (I) to the second operating mode (II), and
- switch from the second operating mode (II) to the first operating mode (I) after the emission of at least one pulse of treatment ultrasound waves.

4. Device (1) according to one of the preceding claims, wherein the device (1) further comprises a manual switch (14) for switching from the first operating mode (I) to the second operating mode (II).

5. Device (1) according to one of the claims 2 to 4, wherein the controller unit (13) is further adapted to switch from the first operating mode (I) to the second operating mode (II) when the detected pressure increase is above a threshold value (TS1).

6. Device (1) according to one of the claims 2 to 4, wherein the controller unit (13) is further adapted to switch from the first operating mode (I) to the second operating mode (II) when the detected pressure increase is above a threshold value (TS1) and then below a second threshold value (TS2) .

7. Device (1) according to one of the claims 2 to 4, wherein the controller unit (13) is further adapted to switch from the second operating mode (II) to the first operating mode (I) when a detected pressure decrease is below a threshold value (TS3).

8. Device (1) according to one of the preceding claims, wherein the device (1) further comprises at least one force sensor (15), preferably a finger force sensor for a device operator, connected to the controller unit (13) and preferably arranged or arrangeable on the object (O), and the controller unit (13) is further adapted to compare a force determined by the force sensor (15) with a threshold value and to switch the device (1) in the second operating mode (II) when the force determined by the force sensor (15) is above the threshold value and is preferably kept constant for a pre-determined amount of time.

9. Device (1) according to claim 8, wherein the controller unit (13) is further adapted to switch from the second operating mode (II) to the first operating mode (I) after the emission of at least one pulse of treatment ultrasound waves when the force determined by the force sensor (15) is below a second threshold value and is preferably kept constant for a pre-determined amount of time.

10. Device (1) according to one of the preceding claims, wherein the controller unit (13) is further adapted to automatically trigger the emission of at least one pulse of treatment ultrasound waves after activation of the second operating mode (II).

11. Device (1) according to one of the claims 1 to 10, wherein the device (1) further comprises a manual switch (16) for triggering the emission of at least one pulse of treatment ultrasound waves by an operator, wherein switching from the first operating mode (I) to the second operating mode (II) is performed automatically after triggering the emission of at least one pulse of treatment ultrasound waves.

12. Device (1) according to claim 11, wherein the device (1) further comprises time measuring means (17) for delaying the emission of at least one pulse of treatment ultrasound waves by a pre-determined amount of time after the second operating mode (II) has been activated.

13. Device (1) according to claim 12, wherein the time measuring means (17) are further adapted to automatically switch from the second operating mode (II) to the first operating mode (I) with a delay of a pre-determined amount of time after emission of the treatment ultrasound waves.

14. Device (1) according to one of the preceding claims, wherein the controller unit (13) is adapted to determine the position of the target (T) based on the images captured by the imaging device (4) and to switch to the second operating mode (II) automatically when the position of the target (T) is stable over a pre-determined amount of time.

15. Device according one of the preceding claims, wherein the device (1) is further adapted to switch to a third operating mode (III), wherein in the third operating mode (III) the compression force applied to the object (O) by the compression unit is maintained at a third compression force value (CF3), preferably higher than the second compression force value (CF2) and in particular the pressure of the coupling liquid is maintained to a third pressure value (P3), preferably higher than the second pressure value (P2), or the volume of the liquid (7) in the cavity (6) is maintained to a third volume value (V3).

## Patentansprüche

1. Ultraschallbehandlungsvorrichtung (1) zur Behandlung von Venen, insbesondere eine HIFU-Behandlungsvorrichtung, wobei die Vorrichtung (1) umfasst:
- einen Kopf (2) mit einem Ultraschallwandler (3) zur Abgabe von Behandlungs-Ultraschallwellen, die auf ein Ziel (T) in einem Objekt (O) fokussiert sind, und eine Bildgebungsvorrichtung (4) zur Abbildung des Objekts (O),
- eine Kompressionsvorrichtung zum Aufbringen einer Kompressionskraft auf das Objekt (O), wobei die Kompressionsvorrichtung entweder durch einen Ballon oder einen Bewegungsmechanismus des Kopfes gebildet wird, und
- eine Steuereinheit (13), die mindestens mit dem Kopf (2)
und der Kompressionsvorrichtung verbunden ist, wobei die Vorrichtung (1) zwischen mindestens zwei Betriebsarten (I, II) umschalten kann, wobei
- in einem ersten Betriebsmodus (I) die von der Kompressionseinheit auf das Objekt (O) ausgeübte Kompressionskraft auf einem ersten Kompressionskraftwert (CF1) gehalten wird, und
- in einem zweiten Betriebsmodus (II) die von der Kompressionseinheit auf das Objekt (O) ausgeübte Kompressionskraft auf einem zweiten Kompressionskraftwert (CF2) gehalten wird, der höher als der erste Kompressionskraftwert (CF1) ist, **dadurch gekennzeichnet, dass** in dem ersten Betriebsmodus (I) die Steuereinheit (13) die Bildgebungsvorrichtung (4) steuert, um eine Bildgebung des Objekts (O) durchzuführen, und in dem zweiten Betriebsmodus (II) die Steuereinheit (13) den Ultraschallwandler (3) steuert, um mindestens einen Impuls von Behandlungsultraschallwellen auszusenden
wobei der zweite Betriebsmodus geeignet ist, eine Vene teilweise, vorzugsweise vollständig, zu kollabieren und/oder den Blutfluss für die Behandlung der Vene zu stoppen.

2. Ultraschallbehandlungsvorrichtung (1) nach Anspruch 1, wobei die Vorrichtung (1) umfasst::
- einen an einer Zielseite des Kopfes (2) angeordneten verformbaren Kopplungsballon (5), wobei ein Hohlraum (6) zwischen dem Kopf (2) und dem Kopplungsballon (5) mit einer Kopplungsflüssigkeit (7) gefüllt oder befüllbar ist und insbesondere in Fluidverbindung mit einem Kopplungsflüssigkeitsreservoir (8) steht
- eine Druckmesseinheit (9) zur Bestimmung des Drucks der Kopplungsflüssigkeit (7) im Hohlraum (6),
- ein Fluidsteuersystem (10) zur Steuerung des Drucks oder des Volumens der Kopplungsflüssigkeit (7) in dem Hohlraum (6), insbesondere mit einer Pumpe (11) und mindestens einem Ventil (12), wobei
- die Steuereinheit (13) mindestens mit der Druckmesseinheit (9), dem Fluidsteuerungssystem (10), dem Kopf (2) und der Kompressionseinheit verbunden ist, und wobei
- im ersten Betriebsmodus (I) der Druck der Kopplungsflüssigkeit (7) auf einem ersten Druckwert (P1) oder auf einem ersten Volumenwert (V1) gehalten wird, und
- im zweiten Betriebsmodus (II) der Druck der Kopplungsflüssigkeit (7) auf einem zweiten Druckwert (P2) gehalten wird, der höher ist als der erste Druckwert (P1), oder das Volumen der Flüssigkeit (7) in dem Hohlraum (6) auf einem zweiten Volumenwert (V2) konstant gehalten wird.

3. Vorrichtung (1) nach Anspruch 2, wobei die Steuereinheit (13) geeignet ist, um:
- den von der mindestens einen Druckmesseinheit (9) ermittelten Druck der Kopplungsflüssigkeit (7) zu überwachen und einen Anstieg des Drucks der Kopplungsflüssigkeit (7) im Hohlraum (6) zu erkennen, wenn sich die Vorrichtung (1) im ersten Betriebsmodus (I) befindet und der Kopplungsballon (5) auf dem Objekt (O) angeordnet ist,
- bei Erkennen eines Schaltsignals vom ersten Betriebsmodus (I) in den zweiten Betriebsmodus (II) zu wechseln,
- nach dem Umschalten vom ersten Betriebsmodus (I) in den zweiten Betriebsmodus (II) die Aussendung mindestens eines Impulses von Behandlungs-Ultraschallwellen auszulösen, und
- Umschalten vom zweiten Betriebsmodus (II) in den ersten Betriebsmodus (I) nach der Aussendung von mindestens einem Impuls von Behandlungsultraschallwellen.

4. Vorrichtung (1) nach einem der vorhergehenden Ansprüche, wobei die Vorrichtung (1) ferner einen manuellen Schalter (14) zum Umschalten vom ersten Betriebsmodus (I) in den zweiten Betriebsmodus (II) aufweist.

5. Vorrichtung (1) nach einem der Ansprüche 2 bis 4, wobei die Steuereinheit (13) ferner dazu ausgebildet ist, von dem ersten Betriebsmodus (I) in den zweiten Betriebsmodus (II) umzuschalten, wenn der erfasste Druckanstieg über einem Schwellenwert (TS1) liegt.

6. Vorrichtung (1) nach einem der Ansprüche 2 bis 4, wobei die Steuereinheit (13) ferner dazu ausgebildet ist, von dem ersten Betriebsmodus (I) in den zweiten Betriebsmodus (II) umzuschalten, wenn der erfasste Druckanstieg oberhalb eines Schwellenwertes (TS1) und dann unterhalb eines zweiten Schwellenwertes (TS2) liegt.

7. Vorrichtung (1) nach einem der Ansprüche 2 bis 4, wobei die Steuereinheit (13) ferner dazu ausgebildet ist, von dem zweiten Betriebsmodus (II) in den ersten Betriebsmodus (I) umzuschalten, wenn ein detektierter Druckabfall unter einem Schwellenwert (TS3) liegt.

8. Vorrichtung (1) nach einem der vorhergehenden Ansprüche, wobei die Vorrichtung (1) ferner mindestens einen Kraftsensor (15), vorzugsweise einen Fingerkraftsensor für einen Gerätebediener, aufweist, der mit der Steuereinheit (13) verbunden und vorzugsweise am Objekt (O) angeordnet oder anordenbar ist, und die Steuereinheit (13) ferner dazu ausgebildet ist, eine von dem Kraftsensor (15) ermittelte Kraft mit einem Schwellenwert zu vergleichen und die Vorrichtung (1) in den zweiten Betriebsmodus (II) zu schalten, wenn die von dem Kraftsensor (15) ermittelte Kraft oberhalb des Schwellenwertes liegt und vorzugsweise für eine vorgegebene Zeitdauer konstant gehalten wird.

9. Vorrichtung (1) nach Anspruch 8, wobei die Steuereinheit (13) ferner dazu eingerichtet ist, nach der Aussendung mindestens eines Impulses von Behandlungs-Ultraschallwellen von dem zweiten Betriebsmodus (II) in den ersten Betriebsmodus (I) umzuschalten, wenn die vom Kraftsensor (15) ermittelte Kraft unter einem zweiten Schwellenwert liegt und vorzugsweise für eine vorgegebene Zeitspanne konstant gehalten wird.

10. Vorrichtung (1) nach einem der vorhergehenden Ansprüche, wobei die Steuereinheit (13) ferner dazu ausgebildet ist, nach Aktivierung des zweiten Betriebsmodus (II) automatisch die Aussendung mindestens eines Pulses von Behandlungsultraschallwellen auszulösen.

11. Vorrichtung (1) nach einem der Ansprüche 1 bis 10, wobei die Vorrichtung (1) ferner einen manuellen Schalter (16) zur Auslösung der Aussendung mindestens eines Impulses von Behandlungsultraschallwellen durch eine Bedienperson aufweist, wobei das Umschalten vom ersten Betriebsmodus (I) in den zweiten Betriebsmodus (II) automatisch nach Auslösung der Aussendung mindestens eines Impulses von Behandlungsultraschallwellen erfolgt.

12. Vorrichtung (1) nach Anspruch 11, wobei die Vorrichtung (1) ferner eine Zeitmesseinrichtung (17) umfasst, um die Aussendung von mindestens einem Impuls von Behandlungs-Ultraschallwellen um eine vorbestimmte Zeitspanne zu verzögern, nachdem der zweite Betriebsmodus (II) aktiviert wurde.

13. Vorrichtung (1) nach Anspruch 12, wobei die Zeitmessmittel (17) ferner so beschaffen sind, dass sie automatisch vom zweiten Betriebsmodus (II) in den ersten Betriebsmodus (I) mit einer Verzögerung von einer vorbestimmten Zeitspanne nach der Emission der Behandlungsultraschallwellen umschalten.

14. Vorrichtung (1) nach einem der vorhergehenden Ansprüche, wobei die Steuereinheit (13) dazu eingerichtet ist, die Position des Ziels (T) anhand der von der Bildgebungsvorrichtung (4) aufgenommenen Bilder zu bestimmen und automatisch in den zweiten Betriebsmodus (II) zu wechseln, wenn die Position des Ziels (T) über eine vorgegebene Zeitspanne stabil ist.

15. Vorrichtung nach einem der vorhergehenden Ansprüche, wobei die Vorrichtung (1) ferner dazu ausgebildet ist, in einen dritten Betriebsmodus (III) zu schalten, wobei in dem dritten Betriebsmodus (III) die von der Kompressionseinheit auf das Objekt (O) aufgebrachte Kompressionskraft auf einem dritten Kompressionskraftwert (CF3) gehalten wird, auf einem dritten Druckkraftwert (CF3), vorzugsweise höher als der zweite Druckkraftwert (CF2), gehalten wird und insbesondere der Druck der Kopplungsflüssigkeit auf einem dritten Druckwert (P3), vorzugsweise höher als der zweite Druckwert (P2), gehalten wird, oder das Volumen der Flüssigkeit (7) in dem Hohlraum (6) auf einem dritten Volumenwert (V3) gehalten wird.

## Revendications

1. Dispositif de traitement par ultrasons (1), pour le traitement des veines, en particulier un dispositif de traitement HIFU, le dispositif (1) comprenant:
- une tête de sonde (2) comprenant un transducteur à ultrasons (3) pour délivrer des ondes ultrasons de traitement focalisées sur une cible (T) à l'intérieur d'un objet (O) et un dispositif d'imagerie (4) pour l'imagerie de l'objet (O),
- un dispositif de compression pour appliquer une force de compression à l'objet (O), le dispositif de compression étant formé par un ballon et un mécanisme de déplacement de la tête de sonde, et
- une unité de commande (13) reliée au moins à la tête de
sonde (2) et à l'unité de compression, dans laquelle le dispositif (1) est adapté pour changer entre au moins deux modes de fonctionnement (I, II), dans lesquels
- dans un premier mode de fonctionnement (I), la force de compression appliquée à l'objet (O) par l'unité de compression est maintenue à une première valeur de force de compression (CF1), et
- dans un deuxième mode de fonctionnement (II), la force de compression appliquée à l'objet (O) par l'unité de compression est maintenue à une deuxième valeur de force de compression (CF2), supérieure à la première valeur de force de compression (CF1), **caractérisé en ce que** dans le premier mode de fonctionnement (I), l'unité de commande (13) commande le dispositif d'imagerie (4) pour réaliser l'imagerie de l'objet (O), et dans le deuxième mode de fonctionnement (II), l'unité de commande (13) commande le transducteur à ultrasons (3) pour émettre au moins une impulsion d'ondes ultrasonores de traitement
dans lequel le second mode de fonctionnement est adapté à la fermeture partielle, de préférence totale, d'une veine et/ou à l'arrêt du flux sanguin pour le traitement de la veine.

2. Dispositif de traitement par ultrasons (1), selon la revendication 1, le dispositif (1) comprenant:
- un ballon de couplage déformable (5) disposé sur un côté cible de la tête de sonde (2), dans lequel une cavité (6) entre la tête de sonde (2) et le ballon de couplage (5) est remplie ou peut être remplie d'un liquide de couplage (7), et est en particulier en liaison fluidique avec un réservoir de liquide de couplage (8)
- une unité de mesure de la pression (9) pour déterminer la pression du liquide de couplage (7) dans la cavité (6),
- un système de contrôle des fluides (10) pour contrôler la pression ou le volume du liquide de couplage (7) dans la cavité (6), comprenant notamment une pompe (11) et au moins une vanne (12), dans lequel
- l'unité de commande (13) est connectée au moins à l'unité de mesure de la pression (9), au système de contrôle des fluides (10), à la tête de sonde (2) et à l'unité de compression, et dans laquelle
- dans le premier mode de fonctionnement (I), la pression du liquide de couplage (7) est maintenue à une première valeur de pression (P1) ou à une première valeur de volume (V1), et
- dans le deuxième mode de fonctionnement (II), la pression du liquide de couplage (7) est maintenue à une deuxième valeur de pression (P2) supérieure à la première valeur de pression (P1) ou le volume du liquide (7) dans la cavité (6) est maintenu constant à une deuxième valeur de volume (V2).

3. Dispositif (1) selon la revendication 2, dans lequel l'unité de commande (13) est adaptée pour:
- surveiller la pression du liquide de couplage (7) déterminée par l'au moins une unité de mesure de la pression (9) et détecter une augmentation de la pression du liquide de couplage (7) dans la cavité (6) lorsque le dispositif (1) est dans le premier mode de fonctionnement (I) et que le ballon de couplage (5) est disposé sur l'objet (O),changer du premier mode de fonctionnement (I) au second mode de fonctionnement (II) sur détection d'un signal de commutation,
- déclencher l'émission d'au moins une impulsion d'ondes ultrasonores de traitement après avoir changé du premier mode de fonctionnement (I) au second mode de fonctionnement (II), et
- changer du deuxième mode de fonctionnement (II) au premier mode de fonctionnement (I) après l'émission d'au moins une impulsion d'ondes ultrasonores de traitement.

4. Dispositif (1) selon l'une des revendications précédentes, dans lequel le dispositif (1) comprend en outre un dispositif de changement manuel (14) pour changer du premier mode de fonctionnement (I) au deuxième mode de fonctionnement (II) .

5. Dispositif (1) selon l'une des revendications 2 à 4, dans lequel l'unité de commande (13) est en outre adaptée pour changer du premier mode de fonctionnement (I) au second mode de fonctionnement (II) lorsque l'augmentation de pression détectée est supérieure à une valeur seuil (TS1)..

6. Dispositif (1) selon l'une des revendications 2 à 4, dans lequel l'unité de commande (13) est en outre adaptée pour changer du premier mode de fonctionnement (I) au second mode de fonctionnement (II) lorsque l'augmentation de pression détectée est supérieure à une valeur seuil (TS1) puis inférieure à une seconde valeur seuil (TS2).

7. Dispositif (1) selon l'une des revendications 2 à 4, dans lequel l'unité de commande (13) est en outre adaptée pour changer du second mode de fonctionnement (II) au premier mode de fonctionnement (I) lorsqu'une baisse de pression détectée est inférieure à une valeur seuil (TS3).

8. Dispositif (1) selon l'une des revendications précédentes, dans lequel le dispositif (1) comprend en outre au moins un capteur de force (15), de préférence un capteur de force du doigt pour un opérateur du dispositif, connecté à l'unité de commande (13) et de préférence disposé ou pouvant être disposé sur l'objet (O), et l'unité de commande (13) est en outre adaptée pour comparer une force déterminée par le capteur de force (15) avec une valeur seuil et pour changer le dispositif (1) dans le deuxième mode de fonctionnement (II) lorsque la force déterminée par le capteur de force (15) est supérieure à la valeur seuil et est de préférence maintenue constante pendant un laps de temps prédéterminé..

9. Dispositif (1) selon la revendication 8, dans lequel l'unité de commande (13) est en outre adaptée pour changer du deuxième mode de fonctionnement (II) au premier mode de fonctionnement (I) après l'émission d'au moins une impulsion d'ondes ultrasons de traitement lorsque la force déterminée par le capteur de force (15) est inférieure à une deuxième valeur seuil et est de préférence maintenue constante pendant une durée prédéterminée.

10. Dispositif (1) selon l'une des revendications précédentes, dans lequel l'unité de commande (13) est en outre adaptée pour déclencher automatiquement l'émission d'au moins une impulsion d'ondes ultrasons de traitement après l'activation du deuxième mode de fonctionnement (II).

11. Dispositif (1) selon l'une des revendications 1 à 10, dans lequel le dispositif (1) comprend en outre un dispositif manuel (16) pour déclencher l'émission d'au moins une impulsion d'ondes ultrasons de traitement par un opérateur, dans lequel le changement du premier mode de fonctionnement (I) au deuxième mode de fonctionnement (II) est effectué automatiquement après le déclenchement de l'émission d'au moins une impulsion d'ondes ultrasons de traitement..

12. Dispositif (1) selon la revendication 11, dans lequel le dispositif (1) comprend en outre des moyens de mesure du temps (17) pour retarder l'émission d'au moins une impulsion d'ondes ultrasons de traitement d'une durée prédéterminée après l'activation du deuxième mode de fonctionnement (II) .

13. Dispositif (1) selon la revendication 12, dans lequel les moyens de mesure du temps (17) sont en outre adaptés pour changer automatiquement du deuxième mode de fonctionnement (II) au premier mode de fonctionnement (I) avec un retard d'une durée prédéterminée après l'émission des ondes ultrasonores de traitement.

14. Dispositif (1) selon l'une des revendications précédentes, dans lequel l'unité de commande (13) est adaptée pour déterminer la position de la cible (T) sur la base des images captées par le dispositif d'imagerie (4) et pour changer automatiquement vers le second mode de fonctionnement (II) lorsque la position de la cible (T) est stable sur une durée prédéterminée.

15. Dispositif selon l'une des revendications précédentes, dans lequel le dispositif (1) est en outre adapté pour changer vers un troisième mode de fonctionnement (III), dans lequel dans le troisième mode de fonctionnement (III) la force de compression appliquée à l'objet (O) par l'unité de compression est maintenue à une troisième valeur de force de compression (CF3), de préférence supérieure à la deuxième valeur de force de compression (CF2) et, en particulier, la pression du liquide de couplage est maintenue à une troisième valeur de pression (P3), de préférence supérieure à la deuxième valeur de pression (P2), ou le volume du liquide (7) dans la cavité (6) est maintenu à une troisième valeur de volume (V3).
